# FASCICULE DE BREVET EUROPEEN

(11) **EP 0 803 491 B1**
(45) Date de publication et mention de la délivrance du brevet: **17.01.2001**
(21) Numéro de dépôt: 97400921.9
(22) Date de dépôt: 23.04.1997
(51) Int. Cl.: C07C 2/30, C07C 11/08

(54) **Procédé amélioré de conversion de l'éthylène en butène-1 avec utilisation d'additifs à base de polyéthylène glycols et de leurs monoéthers et monoesters**
Verbessertes Verfahren zur Umwandlung von Ethylen in 1-Buten unter Verwendung von Additiven die auf Polyethylenglycol und seinen Monoethern und Monoestern basieren
Improved process for the conversion of ethylene to 1-butene using additives which are based on polyethyleneglycol and its monoethers and monoesters

(30) Priorité: 26.04.1996 FR 9605401
(43) Date de publication de la demande: 29.10.1997
(73) Titulaire: INSTITUT FRANCAIS DU PETROLE, 92500 Rueil Malmaison (FR); Saudi Basic Industries Corporation (SABIC), Riyadh 11422 (SA)
(72) Inventeur: Commereuc, Dominique, 92190 Meudon (FR); Hugues, François, 69390 Vernaison (FR); Chauvin, Yves, 78230 Le Pecq (FR); Glaize, Yves, 69360 Saint Symphorien D'Ozon (FR)
(74) Mandataire: Andreeff, François

(56) Documents cités:
- US-A- 4 532 370
- US-A- 4 615 998

## Description

L'objet de la présente invention est un procédé amélioré de synthèse du butène-1 par dimérisation de l'éthylène, grâce à l'utilisation d'additifs à base de polyéthylèneglycols et / ou de certains de leurs dérivés qui sont les monoéthers ou les monoesters de polyéthylèneglycols.

Dans le procédé de dimérisation de l'éthylène en butène-1 tel que décrit dans les brevets US 4 532 370 et 4 615 998, au moyen d'un catalyseur homogène obtenu par l'interaction d'un mélange préformé d'un titanate d'alkyle et d'un éther avec un composé d'aluminium de formule AlR₃ ou AlR₂H, l'éther étant choisi dans le groupe formé par le diéthyléther, le diisopropyléther, le dibutyléther, le méthyl-t-butyléther, le tétrahydrofurane, le 1,4-dioxane, le dihydropyrane, le diméthyléther de l'éthylèneglycol, il se forme de petites quantités de polymère solide qui se déposent et collent sur la surface du réacteur et des tubes d'échangeurs de chaleur et qui sont très nuisibles à la bonne marche du procédé car elles réduisent les transferts de chaleur et nécessitent l'arrêt fréquent du réacteur afin de les éliminer.

Il a maintenant été trouvé que si la réaction de dimérisation est conduite en présence d'additifs composés par des polyéthylèneglycols et / ou certains de leurs dérivés qui sont les monoéthers de polyéthylèneglycols ou les monoesters de polyéthylèneglycols, l'adhérence du polymère aux parois du réacteur et des échangeurs est considérablement diminuée, et la quantité de polymère solide sous-produit est encore réduite.

L'emploi de tels additifs est également possible en l'absence d'éther tel que défini précédemment, plus de polymère est formé mais son adhérence très faible le rend facilement éliminable.

L'invention a ainsi pour objet un procédé amélioré de conversion de l'éthylène en butène-1, dans lequel, dans une enceinte réactionnelle, on met l'éthylène en contact avec une solution d'un catalyseur obtenu par l'interaction d'au moins un titanate d'alkyle avec un composé d'aluminium de formule AlR₃ ou AlR₂H, chacun des restes R étant un radical hydrocarbyle, et en présence d'au moins un additif choisi dans le groupe formé par les polyéthylèneglycols et leurs dérivés.

Les éléments de la solution de catalyseur sont décrits dans les brevets US 4.532.370 et US 4.615.998 dont les enseignements sont ci-inclus.

Les titanates d'alkyle utilisés dans l'invention répondent à la formule générale Ti(OR')₄ dans laquelle R' est un radical alkyle linéaire ou ramifié comportant de préférence de 2 à 8 atomes de carbone. On peut citer par exemple le titanate de tétraéthyle, le titanate de tétraisopropyle, le titanate de tétra-n-butyle, le titanate de tétra-éthyl-2-hexyle.

De façon particulièrement avantageuse, pour augmenter l'activité et la sélectivité de la réaction, la solution de catalyseur résulte de l'interaction d'un mélange préformé d'au moins un titanate d'alkyle et d'au moins un éther, avec au moins un composé d'aluminium tel que défini ci-dessus. Lesdits éthers utilisables peuvent être des monoéthers ou des polyéthers. On peut utiliser par exemple le diéthyléther, le diisopropyléther, le dibutyléther, le méthyl-t-butyléther, le tétrahydrofurane, le dioxane-1,4, le dihydropyrane, l'éther diméthylique de l'éthylèneglycol. Les éthers préférés sont le tétrahydrofurane et / ou le dioxane-1,4. Ils sont utilisés seuls ou en mélange.

Lesdits éthers sont utilisés dans un rapport molaire de 0 à 10, avantageusement de 0,1 à 10 ou de 0,5 à 10, de préférence de 1 à 5, plus particulièrement 2 à 4 moles d'éther par mole de composé du titane. Sans être lié à aucune théorie, on peut penser que l'éther se complexe sur le titane, lui permettant ainsi d'être hexacoordiné. Si on met en oeuvre l'éther dans des rapports supérieurs à 10 moles d'éther par mole de titane, par exemple 20 et au delà, ou s'il est utilisé comme solvant de la réaction, on observe que la réaction est considérablement ralentie et que sa sélectivité est moins bonne, et même, dans certains cas, que la réaction ne se produit plus du tout.

Les composés de l'aluminium utilisés pour préparer le catalyseur sont représentés par la formule générale AlR₃ ou AlR₂H dans laquelle R est un radical hydrocarbyle, de préférence alkyle, comprenant de 2 à 6 atomes de carbone. Le composé AlR₃ est préféré. On peut citer à titre d'exemple le triéthylaluminium, le tripropylaluminium, le tri-iso-butylaluminium, le trihexylaluminium.

Les composants du catalyseur peuvent être mis en contact dans un hydrocarbure et / ou dans le butène-1 produit de dimérisation et / ou dans un, ou les, sous-produit de la réaction tels que les hexènes, de préférence en présence d'éthylène. Le rapport molaire entre le composé de l'aluminium et celui du titane est d'environ 1 : 1 à 20 : 1 et de préférence de environ 2 : 1 à 5 : 1. La concentration du titane dans la solution ainsi préparée est avantageusement comprise entre 10⁻⁴ et 0,5 mole par litre et de préférence entre 2.10⁻³ et 0,1 mole par litre.

La température à laquelle se fait la préparation du catalyseur est habituellement comprise entre -10 et +80 °C, de préférence entre -10 et +45°C. Quand de l'éthylène est présent dans le milieu, sa quantité correspond de préférence à la saturation de la solution à la température considérée et à la pression choisie, 1 bar ou plus. La solution de catalyseur ainsi obtenue peut être utilisée telle quelle ou elle peut être diluée par addition des produits de la réaction.

Les polyéthylèneglycols et leurs monoéthers utilisés selon l'invention répondent à la formule générale H(O-CH₂-CH₂)ₙOR" dans laquelle R" est un atome d'hydrogène ou un radical hydrocarbyle, de préférence alkyle, comprenant de 5 à 30 atomes de carbone, et n est un nombre entier de 4 à 30. Les monoesters de polyéthylèneglycols ont pour formule H(O-CH₂-CH₂)ₙO-(C=O)-R". On peut citer à titre d'exemple les polyéthylèneglycols de masse moléculaire 200 à 10000 ou plus, les monolauryléthers de polyéthylèneglycols, les monostéaryléthers de polyéthylèneglycols, les monolaurates de polyéthylèneglycols, les monostéarates de polyéthylèneglycols. Les polyéthylèneglycols et / ou leurs dérivés peuvent être mis en oeuvre tels quels ou sous forme d'une solution dans un milieu hydrocarboné choisi dans le groupe formé par les hydrocarbures et / ou par le butène-1 produit de dimérisation et / ou par un, ou les, sous-produit de la réaction tels que les hexènes.

Que ce soit un procédé continu ou discontinu, les polyéthylèneglycols et / ou leurs dérivés, purs ou en solution, peuvent être introduits avant de procéder à la réaction de dimérisation de l'éthylène, par exemple ils peuvent être utilisés pour effectuer un traitement de passivation des parois de l'enceinte réactionnelle préalablement au démarrage de la réaction. Les parois de l'enceinte sont métalliques (métaux, aciers, alliages,...) et peuvent avoir subi des traitements de protection (polissage, vitrification,...) ou peuvent avoir été soumises à une protection anodique.

La passivation est effectuée selon toutes les techniques connues. Avantageusement, on charge l'enceinte avec une solution de 20 ppm à 5 % en poids d'additif dans un milieu hydrocarboné, le contact est maintenu de préférence sous agitation pendant 10 min à 10 h, de préférence 30 min à 3 h, à une température inférieure à la température d'ébullition du solvant, 20 à 100° C généralement et 30 à 80 °C de préférence. La solution est ensuite généralement évacuée.

Les polyéthylèneglycols et / ou leurs dérivés, purs ou en solution, peuvent aussi être introduits de façon continue ou en discontinu pendant le déroulement de la réaction, par exemple en mélange avec la solution du titanate, de préférence sous forme d'un flux indépendant des flux de catalyseur. Il peut être avantageux de combiner un traitement de passivation préalable de l'enceinte réactionnelle suivi d'une injection en continu ou en discontinu pendant le déroulement de la réaction.

La quantité de polyéthylèneglycols et / ou de dérivés de polyéthylèneglycols mise en oeuvre pendant la réaction de dimérisation peut représenter de 1 partie par million en poids (ppm) à 5 % en poids, avantageusement 1 ppm à 1 %, de préférence de 20 ppm à 5000 ppm, par rapport au butène-1 produit, que cette quantité soit introduite pendant la réaction (procédé continu) ou dans l'enceinte avant réaction (procédé discontinu).

La réaction de dimérisation de l'éthylène peut être mise en oeuvre à une température de 20 à 150 °C, de préférence de 20 à 70 °C et de manière encore plus préférée de 40 à 70 °C. La pression est de préférence de 0,5 à 8 MPa.

Dans un mode de mise en oeuvre de la réaction catalytique de dimérisation en discontinu, on introduit dans un réacteur (enceinte réactionnelle) muni des habituels systèmes d'agitation et de refroidissement l'additif avec la solution de catalyseur, par exemple un volume choisi de solution catalytique, préparée comme décrit ci-dessus, et, indépendamment, un volume choisi d'une solution de polyéthylèneglycol et / ou de dérivé de polyéthylèneglycol, après quoi on pressurise par de l'éthylène et on ajuste la température à la valeur souhaitée. On alimente le réacteur par de l'éthylène à pression constante jusqu'à ce que le volume total de liquide produit remplisse presque complètement le réacteur. On détruit, après réaction, le catalyseur, par exemple par injection d'eau, d'ammoniac ou d'une amine, et on soutire et sépare les produits de la réaction et les solvants éventuels.

En cas d'opération en continu, on peut avantageusement commencer chaque marche par une passivation des parois du réacteur par un volume choisi d'une solution de polyéthylèneglycol et / ou de dérivé de polyéthylèneglycol. Après avoir soutiré cette solution et avantageusement rincé le réacteur avec un hydrocarbure, la solution catalytique est injectée en continu en même temps qu'une solution de polyéthylèneglycol et / ou de dérivé de polyéthylèneglycol et en même temps que l'éthylène. La température et la pression sont maintenues constantes au moyen de tout système de régulation habituel. L'effluent du réacteur est envoyé dans un système de colonnes à distiller qui permet de séparer d'une part le butène-1 de l'éthylène, qui est renvoyé au réacteur, d'autre part les hexènes et les octènes sous-produits, dont une partie peut être renvoyée dans la section de préparation du catalyseur. Le pied de colonne contenant le catalyseur, les sous-produits lourds et l'additif peut-être incinéré, ou bien le catalyseur récupéré est recyclé.

On a pu constater que la présence d'additif selon l'invention permet d'augmenter de façon significative la durée de marche d'un réacteur en continu, le polymère étant alors, en grande partie, éliminé du réacteur lors du soutirage de l'effluent et/ou par un soutirage en fond du réacteur où il se dépose.

Les exemples suivants illustrent l'invention sans en limiter la portée.

### EXEMPLES 1 A 6

Une série de tests a été effectuée pour déterminer l'effet inhibiteur des polyéthylèneglycols et des dérivés de polyéthylèneglycols sur la formation et l'adhérence aux parois du polymère sous-produit dans la dimérisation de l'éthylène en butène-1. L'effet de la production de polymère est très important en termes d'encrassement des parois du réacteur et des tubes des échangeurs parce qu'il limite les transferts de chaleur indispensables à l'élimination de la chaleur de réaction. Cet effet néfaste est observé même quand la quantité d'éthylène transformé en polymère est très faible en regard de la quantité d'éthylène qui est dimérisé en butène-1.

On utilise pour effectuer la réaction de dimérisation de l'éthylène un autoclave de type Grignard en acier inoxydable d'un volume de 250 ml, muni d'une double enveloppe permettant de réguler la température par une circulation d'eau, et d'un barreau magnétique pour l'agitation.

Dans chaque exemple, le catalyseur est préparé, à une température de 25 °C, en introduisant successivement dans le réacteur sous pression atmosphérique d'éthylène : 25 ml d'heptane, 9,8 ml d'une solution de 0,5 ml de triéthylaluminium dans 19,5 ml d'heptane (soit 1,77 mmole), 2 ml d'une solution à 10 % en volume de titanate de tétra-n-butyle dans l'heptane (soit 0,59 mmole), et une quantité variable selon les essais de l'additif polyéthylèneglycol ou dérivé de polyéthylèneglycol. Après 2 minutes d'interaction, la température est portée à 70 °C et la pression d'éthylène à 2 MPa.

La réaction de dimérisation est arrêtée par injection de 2 ml d'eau lorsque environ 100 litres gazeux (ramenés aux conditions normales) d'éthylène ont été consommés. Le réacteur est ensuite dépressurisé, le gaz étant comptabilisé et recueilli dans un gazomètre pour être analysé. Après ouverture du réacteur, on en collecte le contenu liquide et solide, qui est lavé avec 20 ml d'une solution aqueuse sulfurique à 10 % afin de redissoudre les résidus de catalyseur. Le polymère solide restant est filtré, séché une nuit à l'étuve à 110 °C et pesé.

Les résultats des tests sont présentés dans le tableau 1, où figurent pour chaque essai : la nature de l'additif, la quantité d'additif mis en oeuvre exprimée en parties par million en poids (ppm) par rapport au butène-1 formé, l'activité du catalyseur exprimée par le nombre de moles d'éthylène consommées par heure, la quantité de polymère formé en ppm par rapport à l'éthylène transformé, et l'aspect physique de ce polymère.

L'exemple 1 est un exemple comparatif en l'absence d'additif, qui ne fait pas partie de l'invention. Par comparaison, il est clair que la mise en oeuvre d'un additif constitué par un polyéthylèneglycol ou un dérivé de polyéthylèneglycol a un effet bénéfique sur la quantité de polymère formé d'une part, mais surtout sur son adhérence aux parois d'autre part, qui est nettement plus faible, ce qui facilite son élimination.

**TABLEAU 1**

| N° | Additif | Additif // Butène-1 (ppm) | Activité (mol C₂/h) | Polymère (ppm) | Aspect |
|---|---|---|---|---|---|
| 1 | sans | 0 | 6,0 | 2330 | (a) |
| 2 | Polyglycol 300 | 200 | 6,0 | 1670 | (b) |
| 3 | H(OCH₂CH₂)₂₃OC₁₂H₂₅ | 720 | 6,0 | 1190 | (b) |
| 4 | H(OCH₂CH₂)₂₀OC₁₈H₃₇ | 750 | 5,8 | 1500 | (b) |
| 5 | H(OCH₂CH₂)₄OC₁₂H₂₅ | 225 | 3,4 | 1000 | (b) |
| 6 | H(OCH₂CH₂)₂₃OCOC₁₂H₂₅ | 500 | 5,0 | 1600 | (b) |
| (a) polymère très fortement accroché aux parois, | | | | | |
| (b) polymère en couche mince d'adhérence très faible. | | | | | |

### EXEMPLES 7 ET 8

Dans le même appareillage que pour les exemples 1 à 6, et en utilisant les mêmes conditions opératoires et le même mode opératoire, on met en oeuvre un catalyseur préparé, à une température de 25 °C, en introduisant successivement dans le réacteur sous pression atmosphérique d'éthylène : 25 ml d'heptane, 10 ml d'une solution de triéthylaluminium dans l'heptane représentant 0,9 mmole de triéthylaluminium, et 2 ml d'une solution dans l'heptane d'un mélange de titanate de tétra-n-butyle (0,29 mmole) avec 2 équivalents molaires de tétrahydrofurane. L'exemple 7 est un exemple comparatif en l'absence d'additif et ne fait pas partie de l'invention. Dans l'exemple 8, on introduit dans le réacteur, après le catalyseur, un additif constitué par un éther de polyéthylèneglycol comme indiqué dans le tableau 2. La comparaison de ces deux exemples montre de nouveau l'effet favorable de l'addition d'un dérivé de polyéthylèneglycol, à la fois sur la quantité de polymère et surtout sur son adhérence aux parois.

**TABLEAU 2**

| N° | Additif | Additif / / Butène-1 (ppm) | Activité (mol C₂/h | Polymère (ppm) | Aspect |
|---|---|---|---|---|---|
| 7 | sans | 0 | 4,4 | 45 | (a) |
| 8 | H(OCH₂CH₂)₂₃OC₁₂H₂₅ | 280 | 3,3 | 6 | (b) |
| (a) polymère très fortement accroché aux parois, | | | | | |
| (b) polymère d'adhérence très faible. | | | | | |

## Revendications

1. Procédé de conversion de l'éthylène en butène-1, dans lequel, dans une enceinte réactionnelle, on met l'éthylène en contact avec une solution d'un catalyseur résultant de l'interaction d'au moins un titanate d'alkyle avec au moins un composé d'aluminium de formule AIR₃ ou AlR₂H, chacun des restes R étant un radical hydrocarbyle, procédé caractérisé en ce qu'il se déroule en présence d'au moins un additif choisi dans le groupe formé par les polyéthylèneglycols et leurs dérivés qui sont les monoéthers de polyéthylèneglycols et les monoesters de polyéthylèneglycols.

2. Procédé selon la revendication 1, caractérisé en ce que la solution de catalyseur résulte de l'interaction d'un mélange préformé d'au moins un titanate d'alkyle et d'au moins un éther, avec au moins un composé d'aluminium de formule AIR₃ ou AIR₂H, chacun des restes R étant un radical hydrocarbyle, et l'éther étant choisi dans le groupe formé par le diéthyléther, le diisopropyléther, le dibutyléther, le méthyl-t-butyléther, le tétrahydrofurane, le 1,4-dioxane, le dihydropyrane, le diméthyléther de l'éthylèneglycol,

3. Procédé selon la revendication 2, caractérisé en ce que le rapport molaire entre l'éther et le composé du titane est inférieur ou égal à 10.

4. Procédé selon l'une des revendications précédentes, caractérisé en ce que la concentration du titane dans la solution de catalyseur est comprise entre 10⁻⁴ et 0,5 mole par litre.

5. Procédé selon l'une des revendications précédentes, caractérisé en ce que le rapport molaire entre le composé de l'aluminium et celui du titane est compris entre 1:1 et 20:1.

6. Procédé selon l'une des revendications précédentes, caractérisé en ce que le composé de l'aluminium est AlR₃.

7. Procédé selon l'une des revendications précédentes, caractérisé en ce que l'éther de polyéthylèneglycol porte un radical alkyle.

8. Procédé selon l'une des revendications précédentes, caractérisé en ce que l'additif est choisi dans le groupe formé par les monolauryléthers de polyéthylèneglycols et les monostéaryléthers de polyéthylèneglycols.

9. Procédé selon l'une des revendications précédentes, caractérisé en ce que l'additif est mis en oeuvre sous forme d'une solution dans un milieu hydrocarboné choisi dans le groupe formé par les hydrocarbures, le produit de dimérisation, le ou les sous-produits de réaction, et leurs mélanges.

10. Procédé selon l'une des revendications 1 à 8, caractérisé en ce que l'additif est mis en oeuvre à l'état pur.

11. Procédé selon l'une des revendications précédentes, caractérisé en ce que, avant de procéder à la conversion, l'additif est introduit dans l'enceinte réactionnelle pour effectuer un traitement de passivation des parois de l'enceinte.

12. Procédé selon l'une des revendications précédentes, caractérisé en ce que l'additif est introduit pendant le déroulement de la réaction de conversion, le procédé étant continu.

13. Procédé selon l'une des revendications 1 à 11 caractérisé en ce que, le procédé étant discontinu, l'additif est introduit en mélange avec la solution de titanate.

14. Procédé selon l'une des revendications 1 à 12, caractérisé en ce que l'additif est introduit indépendamment de la solution de catalyseur.

15. Procédé selon l'une des revendications precedentes, caractérisé en ce que la quantité de polyéthylèneglycol et / ou de dérivé de polyéthylèneglycol mise en oeuvre pendant la réaction de conversion est de 1 ppm à 5 % en poids par rapport au butène-1 produit.

16. Procédé selon l'une des revendications précédentes, caractérisé en ce que la quantité de polyéthylèneglycol et / ou de dérivé de polyéthylèneglycol mise en oeuvre pendant la réaction de conversion est de 1 ppm à 1 % en poids par rapport au butène-1 produit.

17. Procédé selon l'une des revendications précédentes, caractérisé en ce que 15 la quantité de polyéthylèneglycol et/ou de dérivé de polyéthylèneglycol mise en oeuvre pendant la réaction de conversion est de 20 ppm à 5000 ppm par rapport au butène-1 produit.

18. Procédé selon l'une des revendications précédentes, caractérisé en ce que 20 le procédé se déroule à une température comprise entre 20 et 150 °C sous une pression de 0,5 à 8 MPa.

## Patentansprüche

1. Verfahren zur Umwandlung von Ethylen in 1-Buten, in dem man in einem Reaktionsgefäß Ethylen mit einer Lösung eines Katalysators in Kontakt bringt, der aus der Wechselwirkung von wenigstens einem Alkyltitanat mit wenigstens einer Aluminiumverbindung der Formel AlR₃ oder AlR₂H resultiert, wobei jeder der Reste R ein Hydrocarbylradikal ist und das Verfahren dadurch gekennzeichnet ist, dass es in Anwesenheit von wenigstens einem Zusatz abläuft, der aus der durch die Polyethylenglykole und ihrer Derivate gebildeten Gruppe, welche Monoether von Polyethylenglykolen und Monoester Polyethylenglykolen sind, gebildet wird.

2. Verfahren nach Anspruch 1, dadurch gekennzeichnet, dass die Katalysatorlösung aus der Wechselwirkung einer vorgebildeten Mischung von wenigstens einem Alkyltitant und von wenigstens einem Ether mit wenigstens einer Aluminuimverbindung der Formel AlR₃ oder AlR₂H resultiert, wobei jeder der Reste R ein Hydrocarbylradikal ist und der Ether aus der durch Diethylether, Diisopropylether, Dibutylether, Methyl-t-Butylether, Tetrahydrofuran, 1,4-Dioxan, Dihydropyran, Dimethylether von Ethylenglykol gebildeten Gruppe gewählt ist.

3. Verfahren nach Anspruch 2, dadurch gekennzeichnet, dass das molare Verhältnis zwischen dem Ether und der Titanverbindung kleiner oder gleich 10 ist.

4. Verfahren nach einem der vorhergehenden Ansprüche, dadurch gekennzeichnet, dass die Konzentration des Titans in der Katalysatorlösung zwischen 10⁻⁴ und 0,5 mol/l liegt.

5. Verfahren nach einem der vorhergehenden Ansprüche, dadurch gekennzeichnet, dass das molare Verhältnis zwischen der Aluminiumverbindung und jener des Titans zwischen 1:1 und 20:1 liegt.

6. Verfahren nach einem der vorhergehenden Ansprüche, dadurch gekennzeichnet, dass die Aluminiumverbindung AlR3 ist.

7. Verfahren nach einem der vorhergehenden Ansprüche, dadurch gekennzeichnet, dass der Polyethylenglykolether ein Alkylradikal trägt.

8. Verfahren nach einem der vorhergehenden Ansprüche, dadurch gekennzeichnet, dass der Zusatz aus der durch die Monolaurylether von Polyethylenglykolen und die Monostearylether von Polyethylenglykolen gebildeten Gruppe gewählt ist.

9. Verfahren nach einem der vorhergehenden Ansprüche, dadurch gekennzeichnet, dass der Zusatz in Form einer Lösung in einem kohlenwasserstoffhaltigen Milieu, gewählt aus der durch die Kohlenwasserstoffe, das Dimerisierungsprodukt, das oder die Nebenprodukte der Reaktion und ihrer Mischung gebildeten Gruppe eingesetzt wird.

10. Verfahren einem der Ansprüche 1 bis 8, dadurch gekennzeichnet, dass der Zusatz in reinem Zustand eingesetzt wird.

11. Verfahren nach einem der vorhergehenden Ansprüche, dadurch gekennzeichnet, dass der Zusatz in das Reaktionsgefäß zum Durchführen einer Passivierungsbehandlung der Wände des Reaktionsgefäßes eingeführt wird, bevor man zu der Umwandlung übergeht.

12. Verfahren nach einem der vorhergehenden Ansprüche, dadurch gekennzeichnet, dass der Zusatz während des Ablaufs der Umwandlungsreaktion eingeführt wird, wobei das Verfahren kontinuierlich ist.

13. Verfahren nach einem der Ansprüche 1 bis 11, dadurch gekennzeichnet, dass der Zusatz im Gemisch mit der Titanatlösung eingeführt wird, wobei das Verfahren diskontinuierlich ist.

14. Verfahren nach einem der Ansprüche 1 bis 12, dadurch gekennzeichnet, dass der Zusatz unabhängig von der Katalysatorlösung eingeführt wird.

15. Verfahren nach einem der vorhergehenden Ansprüche, dadurch gekennzeichnet, dass die Menge an Polyethylenglykol und/oder an Derivat von Polyethylenglykol, die während der Unwandlungsreaktion eingesetzt wird, von 1 ppm bis 5 Gew.-% im Verhältnis zum hergestellten 1-Buten beträgt.

16. Verfahren nach einem der vorhergehenden Ansprüche, dadurch gekennzeichnet, dass die Menge an Polyethylenglykol und/oder an Derivat von Polyethylenglykol, die während der Umwandlungsreaktion eingesetzt wird, von 1 ppm bis 1 Gew.-% im Verhältnis zum hergestellten 1-Buten beträgt.

17. Verfahren nach einem der vorhergehenden Ansprüche, dadurch gekennzeichnet, dass die Menge an Polyethylenglykol und/oder an Derivat von Polyethylenglykol, die während der Umwandlungsreaktion eingesetzt wird, von 20 ppm bis 5000 ppm im Verhältnis zum hergestellten 1-Buten beträgt.

18. Verfahren nach einem der vorhergehenden Ansprüche, dadurch gekennzeichnet, dass das Verfahren bei einer Temperatur, die zwischen 20 und 150°C liegt, und unter einem Druck von 0,5 bis 8 MPa abläuft.

## Claims

1. A process for the conversion of ethylene to but-1-ene, wherein, in a reaction enclosure, the ethylene is brought into contact with a solution of a catalyst resulting from the interaction of at least one alkyl titanate with at least one aluminium compound of the formula AlR₃ or AlR₂H, each of the residues R being a hydrocarbyl radical, the process being characterised in that it takes place in the presence of at least one additive selected from the group formed by polyethylene glycols, and dérivatives thereof which are monoethers of polyethylene glycols and monoesters of polyethylene glycols.

2. A process according to claim 1 characterised in that the catalyst solution results from the interaction of a preformed mixture of at least one alkyl titanate and at least one ether, with at least one aluminium compound of the formula AIR₃ or AIR₂H, each of the residues being a hydrocarbyl radical, and the ether being selected from the group formed by diethylether, diisopropylether, dibutylether, methyl-t-butylether, tetrahydrofuran, 1-4-dioxan, dihydropyran, and ethylene glycol dimethylether.

3. A process according to claim 2 characterised in that the moral ratio between the ether and the titanium compound is less than or equal to 10.

4. A process according to one of the preceding claims characterised in that the concentration of titanium in the catalyst solution is between 10⁻⁴ and 0.5 mole per litre.

5. A process according to one of the preceding claims characterised in that the moral ratio between the aluminium compound and the titanium compound is between 1:1 and 20:1.

6. A process according to one of the preceding claims characterised in that the aluminium compound is AlR₃.

7. A process according to one of the preceding claims characterised in that the polyethylene glycol ether bears an alkyl radical.

8. A process according to one of the preceding claims characterised en that the additive is selected from the group formed by monolaurylethers of polyethylene glycols and monostearylethers of polyethylene glycols.

9. A process according to one of the preceding claims characterised in that the additive is used in the form of a solution in a hydrocarbon medium selected from the group formed by hydrocarbons, the dimerisation product, the reaction by-product or by-products and mixtures thereof.

10. A process according to one of claims 1 to 8 characterised in that the additive is used in the pure state.

11. A process according to one of the preceding claims characterised in that, before proceeding with the conversion operation, the additive is introduced into the reaction enclosure to effect a treatment for passivation of the walls of the enclosure.

12. A process according to one of the preceding claims characterised in that the additive is introduced while the conversion reaction is taking place, the process being continuous.

13. A process according to one of claims 1 to 11 characterised in that, the process being discontinuous, the additive is introduced into the reaction enclosure with the catalyst solution.

14. A process according to one of the claims 1 to 12 characterised in that the additive is introduced independently of the catalyst solution.

15. A process according to one of the preceding claims characterised in that the amount of polyethylene glycol and/or polyethylene glycol dérivative used during the conversion reaction is from 1 ppm to 5% by weight with respect to the but-1-ene produced.

16. A process according to one of the preceding claims characterised in that the amount of polyethylene glycol and/or polyethylene glycol dérivative used during the conversion reaction is from 1 ppm to 1% by weight with respect to the but-1-ene produced.

17. A process according to one of the preceding claims characterised in that the amount of polyethylene glycol and/or polyethylene glycol dérivative used during the conversion reaction is from 20 ppm to 5000 ppm with respect to the but-1-ene produced.

18. A process according to one of the preceding claims characterised in that the process takes place at a temperature of between 20 and 150°C under a pressure of from 0.5 to 8 Mpa.
